# EUROPÄISCHE PATENTSCHRIFT

(11) **EP 1 813 589 B1**
(45) Veröffentlichungstag und Bekanntmachung des Hinweises auf die Patenterteilung: **15.04.2009**
(21) Anmeldenummer: 07000853.7
(22) Anmeldetag: 17.01.2007
(51) Int. Cl.: C07C 45/30, C07C 45/34, C07C 47/06

(54) **Verfahren zur Herstellung von Acetyldehyd aus Ethylen und Sauerstoff**
Method for producing acetyldehyde from ethylene and oxygen
Procédé destiné à la fabrication d'acétyldéhyde à partir d'éthylène et d'oxygène

(30) Priorität: 28.01.2006 DE 10604074
(43) Veröffentlichungstag der Anmeldung: 01.08.2007
(73) Patentinhaber: Celanese Chemicals Europe GmbH, 61476 Kronberg (DE)
(72) Erfinder: Franken-Stellamans, Erhard, Dr., 65527 Niedernhausen (DE); Fähtz, Mario, 65606 Vilmar (DE); Hett, Klaus, 61350 Bad Homburg (DE)

(56) Entgegenhaltungen:
- DE-A1- 2 521 092
- US-A1- 2002 087 032

## Beschreibung

Die vorliegende Erfindung betrifft ein einstufiges Verfahren zur Oxidation von Ethylen zu Acetaldehyd in Gegenwart einer wässrigen Katalysatorlösung mittels einer verbesserten Wärmeisolierung in einem Schlaufenreaktor.

Es ist bekannt, Ethylen oder andere Olefine, wie Propylen, Butylen oder Isobutylen mit Sauerstoff oder Sauerstoff enthaltenden Gasen zu Aldehyden, Ketonen oder den Aldehyden entsprechenden Säuren zu oxidieren. Die Umsetzung erfolgt in Wasser in Gegenwart von Edelmetallen der Gruppen VIII des Periodensystems der Elemente oder deren Salzen, die mit den Olefinen Komplexverbindungen bilden, und Redoxsystemen (DE 11 90 451).

Großtechnische Bedeutung hat die Oxidation von Ethylen zu Acetaldehyd erlangt. Zur Katalyse der stark exothermen Oxidationsreaktion mit Sauerstoff als Oxidationsmittel wird üblicherweise eine salzsaure, wässrige Lösung von Kupferchlorid und Palladiumchlorid verwendet. Das Reaktionsgeschehen lässt sich prinzipiell in zwei Teilschritte untergliedern. In der sogenannten Ethylenreaktion erfolgt die Acetaldehydbildung aus Ethylen und Wasser in homogener Katalyse in Gegenwart eines Palladium(II)-chlorokomplexes, der dabei unter Bildung von elementarem Palladium zerfällt, das durch ebenfalls anwesendes Kupfer(II)-chlorid wieder zu Palladium mit der Oxidationsstufe +II oxidiert wird. Das dabei gebildete Kupfer(I)-chlorid wird dann in der sogenannten Oxidationsreaktion mit Sauerstoff in salzsaurer Lösung zum Kupfer(II)-chlorid oxidiert, das dann wieder in der Ethylenreaktion aktiv ist.

Bei der technischen Ausgestaltung der Acetaldehydherstellung haben sich zwei Verfahrensvarianten entwickelt. Bei dem einstufigen Verfahren erfolgt die Ethylenreaktion und die Oxidationsreaktion im selben Reaktor, während nach dem zweistufigen Verfahren die Ethylenreaktion und die Oxidationsreaktion in getrennten Reaktoren ablaufen. Diese zweistufige Verfahrensvariante benötigt jedoch eine energieaufwändige Katalyatorumwälzung und ist technischer weniger häufig realisiert worden als die Einstufen-Variante. Die bei der einstufigen Verfahrensvariante in einem Reaktorgefäß ablaufende Ethylenreaktion und Oxidationsreaktion wird im Folgenden mit dem Begriff "Ethylenoxidation" zusammengefasst, um die Acetaldehydbildung in dem Reaktiosgefäß zu beschreiben.

Bei dem einstufigen Verfahren hat sich ein sogenannter Schlaufenreaktor als zweckmäßiges Reaktionsgefäß erwiesen. Schlaufenreaktoren sind in der chemischen Prozesstechnik übliche Reaktionsgefäße. Sie zählen zu den statischen Reaktionsgefäßen und in ihnen findet die Durchmischung mit Rückvermischung aufgrund der durch die Reaktionswärme verursachten Konvektionsströmung statt. Der bei der technischen Herstellung von Acetaldehyd durch Ethylenoxidation verwendete Schlaufenreaktor kann prinzipiell als Anordnung von zwei über Rohrleitungen miteinander verbundenen Reaktionskesseln beschrieben werden, wobei ein Reaktionskessel als Reaktor und der andere als Nebelfänger arbeitet. Zwischen Reaktor und Nebelfänger wird ein flüssiger Katalysator-Zwangsumlauf aufrechterhalten. Die hierzu benötigte Energie wird zum einen aus der Reaktionswärme der stark exothermen Ethylenoxidation geliefert und zum anderen treiben die in den Reaktor eingespeisten Gase Ethylen, Sauerstoff und zurückgeführtes Kreisgas ebenfalls den Katalysator-Zwangsumlauf an. Die Reaktion wird unter Druck und am Siedepunkt der Reaktionsmischung durchgeführt, wobei der Druck über die Ethyleneinspeisung je nach Anlagenauslastung eingestellt wird. In Abhängigkeit von dem vorgegebenen Druck stellt sich die zugehörige Siedetemperatur und damit auch die Reaktionstemperatur im Reaktor ein. Die Reaktionswärme wird für den Antrieb des Katalysator-Zwangsumlaufs genutzt und darüber hinaus durch Verdampfen von Acetaldehyd und Wasser abgeführt. Der Reaktor wird somit unter Siedekühlung betrieben. Neben dem zwischen dem Reaktor und Nebelfänger stattfindenden Katalysator-Zwangsumlauf wird auch im Reaktor selbst durch Konvektionsströmung ein Katalysatorzwangs-Umlauf aufrechterhalten.

Das entstehende Reaktionsgemisch, enthaltend im Wesentlichen die wässrige Katalysatorlösung, Wasserdampf, gasförmigen Acetaldehyd und Nebel aus der wässrigen Katalysatorlösung strömt in den Nebelfänger über, in dem die Acetaldehyd haltigen Prozessgase verdampfen. Die wässrige Katalysatorlösung sowie kondensierter Wasserdampf und -nebel wird anschließend wieder in den Reaktor zurückgeführt. Treibende Kräfte hierfür sind, wie bereits beschrieben, die Reaktionswärme beziehungsweise die kinetische Energie der eingespeisten Gase.

Es wurde nun überraschenderweise gefunden, dass sich die Katalysator-Selektivität und damit die Raum-Zeit-Ausbeute in der Ethylenoxidation verbessern lässt, wenn man die Katalysatorumwälzung in dem Schlaufenreaktor erhöht. Eine Verbesserung der Raum-Zeit-Ausbeute führt zu einer Kapazitätserhöhung der bestehenden Acetaldehyd-Produktions-Anlage ohne zusätzliche aufwändige Investionskosten oder bedeutet, bei gleichbleibender Produktionsleistung, einen niedrigeren Systemdruck und damit verbunden eine niedrigere Reaktionstemperatur. Eine niedrigere Reaktionstemperatur geht dann aber mit einer verbesserten Katalysator-Selektivität einher, so dass weniger gasförmige Nebenprodukte, beispielsweise Kohlendioxid, wie auch weniger wasserlösliche Nebenprodukte, beispielsweise Essigsäure, anfallen.

Die vorliegende Erfindung betrifft daher ein einstufiges Verfahren zur Oxidation von Ethylen zu Acetaldehyd in Gegenwart einer wässrigen Katalysatorlösung, die aus einer Lösung von Kupferchlorid und Palladiumchlorid besteht, unter Aufrechterhaltung der Umwälzung der wässrigen Katalysatorlösung in einem Schlaufenreaktor, der aus einem Reaktor und einem Nebelfänger aufgebaut ist, dadurch gekennzeichnet, dass der gesamte Schlaufenreaktor eine von außen aufgebrachte Wärmeisolierung aufweist.

Überraschenderweise kann durch eine deutliche Erhöhung der Wärmeisolierung des Schlaufenreaktors die Raum-Zeit-Ausbeute an Acetaldehyd in der Ethylenoxidation verbessert werden. Ohne auf mechanistische Überlegungen eingehen zu wollen, kann vermutet werden, dass eine verbesserte Wärmeisolierung die Umwälzungsgeschwindigkeit der wässrigen Katalysatorlösung erhöht und so höhere stationäre Konzentrationen an Kupfer(II)-chlorid in der Ethylenumsetzungszone des Schlaufenreaktors sicherstellt. Eine höhere Konzentration an Kupfer(II)-chlorid bewirkt wiederum eine höhere Konzentration an Palladium(II)-Komplexen, an denen die eigentliche Umsetzung von Ethylen und Wasser zu Acetaldehyd stattfindet.

Als mögliche Maßnahme, die Umwälzungsgeschwindigkeit der wässrigen Katalysatorlösung in dem Schlaufenreaktor zu erhöhen, kann eine Erhöhung der Kreisgasmenge in Erwägung gezogen werden. Unter Kreisgas versteht man den bei der Aufarbeitung des Reaktionsgemisches zu rohem Acetaldehyd anfallenden Gasstrom, der nach Abtrennung von Acetaldehyd und Ausschleusung eines Inerte enthaltenden Abgasstromes wieder in den Reaktorteil zurückgeführt wird. Eine Erhöhung der Kreisgasmenge bewirkt zwar auf der einen Seite eine Erhöhung der Umwälzungsgeschwindigkeit der wässrigen Katalysatorlösung in dem Schlaufenreaktor, sie ist jedoch mit vielen Nachteilen behaftet. Eine erhöhte Kreisgasfahrweise führt zu einer verstärkten Belastung des Kreisgasverdichters, was mit einem höheren Aufwand an Kompressionsenergie verbunden ist. Ebenfalls muss in dem Kreisgaswäscher bei verstärkter Belastung auch die Menge an Waschwasser erhöht werden, das sich im rohen Acetaldehyd ansammelt und energetisch aufwändig wieder abgetrennt werden muss. Eine Erhöhung der Kreisgasmenge führt auch zu einem verstärkten Tröpfchen-Austrag an wässriger Katalysatorlösung aus dem Reaktor und damit zu Edelmetallverlusten. Schließlich führt der verstärkte Eintrag an dem gekühlten Kreisgas auch zu einem Entzug von Reaktionswärme, da das gekühlte und eingetragene Kreisgas wieder aufgeheizt werden muss.

Als entscheidende und überraschende Maßnahme, die Umwälzungsgeschwindigkeit der wässrigen Katalysatorlösung in dem Schlaufenreaktor zu erhöhen, hat sich nun eine verbesserte Wärmeisolierung dieses Schlaufenreaktors erwiesen. Dadurch wird der Wärmeverlust aufgrund der Eigenkonvektion der den Schlaufenreaktor umgebenden und bewegten Luft und aufgrund der Wärmeabstrahlung von der Reaktoroberfläche verringert. Infolge der verbesserten Wärmeisolierung verbleibt ein höherer Anteil der freigesetzten Reaktionswärme in dem Reaktionssystem und führt zu einer verstärkten Verdampfung des Wassers, das als Lösungsmittel für die Kupfer- und Palladiumsalze dient. Eine höhere Verdampfungsrate an Wasser führt zu einer Vergrößerung der Dichteunterschiede zwischen dem Reaktorinhalt und dem Nebelfängerinhalt, woraus sich dann eine Steigerung der Umwälzungsgeschwindigkeit der wässrigen Katalysatorlösung ergibt. Nach der erfindungsgemäßen Arbeitsweise wird somit die Reaktionswärme der stark exothermen Ethylenoxidationsreaktion über die verbesserte Katalysatorumwälzung letztendlich für eine Selektivitätsverbesserung für die zum gewünschten Acetaldehyd führende Reaktion genutzt.

Als weiteren Effekt, der sich infolge der verbesserten Wärmeisolierung des Schlaufenreaktors einstellt, beobachtet man einen rascheren Austrag des gebildeten Acetaldehyds aus der Reaktionszone infolge verstärkter Verdampfung. Dadurch vermindert sich die stationäre Konzentration an Acetaldehyd im Reaktionsgemisch und die Bildung von Nebenprodukten, die auf der unerwünschten Weiterreaktion des gebildeten Acetaldehyds in dem Reaktor basieren, kann reduziert werden.

Bei der Wahl geeigneter Materialien für den Schlaufenreaktor, die eine Verbesserung der Wärmeisolierung zulassen, müssen sowohl die aggressiven Eigenschaften der wässrigen Katalysatorlösung sowie die Temperaturbealstung aufgrund der hohen Reaktionswärme, die im Allgemeinen in einem Bereich von 110-140°C liegt, berücksichtigt werden. Die wässrige Katalysatorlösung weist aufgrund ihres Gehalts an Salzsäure sowie aufgrund der gelösten Palladium- und Kupferchloride gegenüber den metallischen Apparatematerialien extrem korrosive Eigenschaften auf.

Die bei der Durchführung des einstufigen Verfahrens zur Acetaldehydbildung konventionell eingesetzten Schlaufenreaktoren, die aus einem Reaktorteil und einem Nebelfänger aufgebaut sind, sind aus einem in der chemischen Apparatetechnik üblichen C-Stahl gefertigt, der zum Zwecke des Korrosionsschutzes innenseitig gummiert ist. Um die Gummiauskleidung vor den hohen Reaktionstemperaturen zu schützen, ist auf dieser Gummiauskleidung eine mehrlagige, säurefeste, keramische Auskleidung aufgebracht. Da diese säurefeste, keramische Auskleidung eine relativ hohe Wärmeleitfähigkeit aufweist, resultiert zwangsläufig ein erheblicher Wärmeverlust und Wärmeabfluss durch die Wandung des gesamten Schlaufenreaktors nach außen hin. Konventionelle Schlaufenreaktoren weisen daher auch eine relativ hohe äußere Oberflächentemperatur von etwa 70°C auf. Durch diese Auslegung des Reaktors wird somit über die Behälterwandung her gesehen ein Temperaturgefälle ausgebildet, das allerdings auch erwünscht ist, um sicherzustellen, dass die Gummiauskleidung nicht über ihre Zersetzungstemperatur von etwa 80°C erhitzt wird.

Die für die Durchführung des erfindungsgemäßen Verfahrens wesentlich verbesserte Wärmeisolierung kann bei Schlaufenreaktoren konventioneller Auslegung nicht erreicht werden, weil eine Verbesserung der Innenisolierung des Reaktors den Einsatz eines Materials erfordert, das sowohl säurefest als auch stark wärmedämmend ist und auf der Gummiauskleidung aufgebracht werden kann. Derartige Materialien stehen kostengünstig nicht zur Verfügung, und ihr Einsatz als Reaktormaterial würde die Investitionskosten derart erhöhen, dass der Acetaldehydherstellungsprozess nicht mehr wirtschaftlich zu betreiben ist. Auch das Aufbringen einer wirksamen Außenisolierung ist bei konventionellen Schlaufenreaktoren nicht möglich, da diese Maßnahme zu einer Überhitzung der innenseitig aufgebrachten Gummilage führt.

Das erfindungsgemäße Verfahren zur Acetaldehydherstellung ist durch die Durchführung in einem Schlaufenreaktor charakterisiert, der aus einem Material oder mehreren Materialschichten gefertigt ist, das eine effektive äußere Wärmeisolierung ermöglicht.

In einer Ausführungsform des erfindungsgemäßen Verfahrens kann der Druck tragende Teil des Schlaufenreaktors aus einem Material gefertigt werden, das gegenüber der wässrigen Katalysatorlösung korrosionsbeständig ist, beispielsweise aus Titan, Tantal oder Niob-Zirkon-Legierungen, vorzugsweise aus Titan.

In einer weiteren Ausführung des erfindungsgemäßen Verfahrens ist für den Druck tragenden Teil des Schlaufenreaktors auch die Verwendung konventioneller, nicht korrosionsbeständiger Materialien, wie zum Beispiel C-Stahl oder üblicher Edelstähle, möglich, sofern die Reaktorinnenwand mit einem korrosionsstabilen Material beschichtet ist, das zugleich eine ausreichende Temperaturbeständigkeit bei Reaktionstemperatur aufweist. Für solche innenseitig aufgebrachte Beschichtungen eignen sich beispielsweise Beschichtungen aus Email oder aus speziellen polyhalogenierten Kohlenwasserstoffen.

Die auf den Schlaufenreaktor von außen aufgebrachte Wärmeisolierung besteht aus einem zur Wärmeisolierung üblichen Material mit einer ausreichenden Temperaturbeständigkeit bei der Reaktionstemperatur der Acetaldehydbildung. Beispiele für solche wärmeisolierende Materialien sind Mineralfasern oder Glasschaum. Die äußere Wärmeisolierung wird zweckmäßigerweise in einer solchen Dicke aufgetragen, dass sich die Temperatur auf der Reaktoroberfläche der Umgebungstemperatur angleicht. Im Allgemeinen wird das zur äußeren Wärmeisolierung verwendete Material in einer Dicke von bis zu 100 Millimetern aufgetragen. Eine darüber hinausgehende weitere Auftragung von wärmeisolierenden Materialien bewirkt keine weitere Verbesserung und verteuert unnötig das Verfahren.

Durch die verbesserte Wärmeisolierung wird die Katalysatorumwälzung im Schlaufenreaktor verbessert, wodurch eine höhere Konzentration an katalytisch aktiven Metallsalzen in der Ethylenumsetzungszone eingestellt werden kann. Dadurch lässt sich wiederum die Raum-Zeit-Ausbeute an Acetaldehyd verbessern. Ebenfalls verdampft infolge der verbesserten Wärmeisolierung der gebildete Acetaldehyd rascher aus der heißen Reaktionszone und die Bildung von Nebenprodukten kann reduziert werden.

Je nach Anlagenlast, d.h. je nach stündlich in den Schlaufenreaktor eingebrachter Menge an Ethylen, lässt sich der spezifische Abgasanfall je Tonne Ethylen reduzieren. Mit einer Verringerung des Abgasanfalls, die im Wesentlichen durch eine geringe Kohlendioxidbildung aufgrund der verbesserten Katalysatorselektivität bedingt ist, geht auch die Verringerung der Bildungsraten anderer Nebenprodukte, zum Beispiel von Essigsäure, einher, die über das Abwasser aus dem Prozess ausgeschleust werden. Damit kann auch die Belastung des ausgeschleusten Abwassers mit organischen Verunreinigungen reduziert werden. Die verbesserte Katalysatorselektivität ermöglicht eine Erhöhung der Anlagenkapazität bei bisher gleich bleibenden Reaktionsbedingungen, oder falls eine bestimmte Produktionsleistung oder die Maximallast gefahren werden soll, die Durchführung der Umsetzung bei vermindertem Anlagendruck. Der mit diesen milden Reaktionsbedingungen verbundene Selektivitätsgewinn führt wegen des geringeren Anfalls an Nebenprodukten zu einer signifikanten Ausbeutesteigerung an Acetaldehyd.

Als einen weiteren Effekt beobachtet man, dass der Gehalt an Kupfer(I)-chlorid am Gesamtkupfergehalt in der wässrigen Katalysatorlösung kaum noch von der Ethylenaufnahme abhängt und sich auf deutlich niedrigerem Niveau bewegt. Durch die verbesserte Wärmeisolierung des Schlaufenreaktors und der damit bedingten verbesserten Katalysatorwälzung wird bewirkt, dass das im Zuge der Reaktion entstehende Kupfer(I)-chlorid sehr rasch wieder in den Bereich der Sauerstoffeinspeisung transportiert und dort wieder zum Kupfer(II)-chlorid oxidiert wird. Die Ethylenaufnahme, die der Anlagenleistung entspricht, ist daher nur noch von geringem Einfluss auf die stationäre Konzentration von Kupfer(I)- und Kupfer(II)-chlorid im gesamten Reaktionssystem.

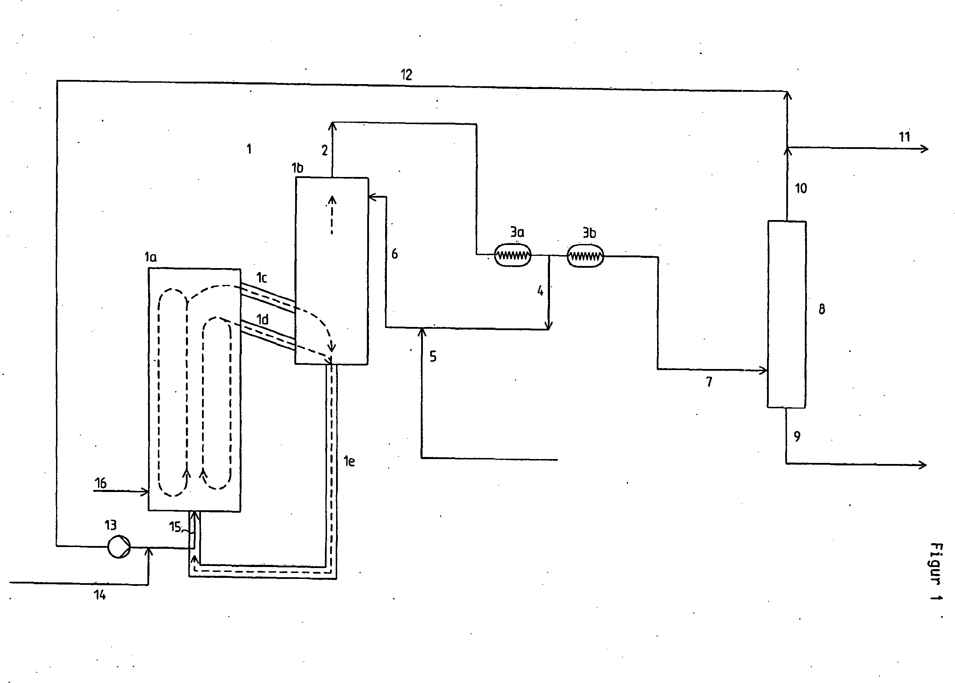

Im Folgenden wird eine beispielhafte Ausgestaltung der Erfindung anhand des in der Figur dargestellten Verfahrensbildes und anhand von Betriebserfahrungen näher beschrieben. Eine Beschränkung der Erfindung in irgendeinerweise ist dadurch nicht beabsichtigt.

In einem aus Titan gefertigten Schlaufenreaktor 1, bestehend aus Reaktor 1a und aus Nebelfänger 1b, wird über die Verbindungsleitungen 1 c und 1 d das siedende Gas-/Flüssigkeitsgemisch aus dem Reaktor 1 a in den Nebelfänger 1b überführt, aus dem über den Boden über die Leitung 1 e die wässrige Katalysatorlösung zurück in den Reaktor 1 a fließt. Der aus dem Nebelfänger 1b verdampfende und gasförmige Produktstrom wird über die Leitung 2 abgeführt und in den Wärmetauschern 3a und 3b abgekühlt, wobei es zur Kondensation kommt. Das nach dem Wärmetauscher 3a anfallende Kondensat wird über die Leitung 4 abgenommen und mit über Leitung 5 herangeführtem Ergänzungswasser abgemischt. Die Mischung wird über Leitung 6 wieder auf den Kopf des Nebelfängers gegeben, die dort im Gegenstrom dem aus der flüssigen Reaktionsmischung verdampfenden Produktstrom entgegenströmt.

Der nach dem Wärmetauscher 3b anfallende Gas- und Kondensatstrom wird über die Leitung 7 auf den unteren Teil einer Waschkolonne 8 gegeben, aus der über Leitung 9 roher Acetaldehyd abgeführt und nach an sich bekannten Verfahren aufgearbeitet wird. Aus dem am Kopf der Waschkolonne über Leitung 10 entnommenen gasförmigen Produkt wird über die Leitung 11 ein unter anderem Inerte enthaltender Abgasstrom ausgeschleust, während der Rest als Kreisgas über die Leitung 12 zurückgeführt, in dem Verdichter 13 aufkomprimiert und mit über Leitung 14 herangeführtem frischen Ethylen abgemischt wird. Das Gasgemisch wird anschließend über Leitung 15 an der Stelle in den Boden des Reaktors 1a eingespeist, an der auch die über die Leitung 1 e herangeführte wässrige Katalysatorlösung in den Reaktor 1 a eintritt. Über Leitung 16 wird frischer Sauerstoff in den Boden des Reaktors 1 a eingedüst. Man kann daher den unteren Teil des Reaktors 1a als Oxidationszone für die Reoxidation von Kupfer(I)- zu Kupfer(II)-chlorid ansehen. Die in dem Reaktor 1 a stattfindende Konvektionsströmung sowie der im Nebelfänger 1b und in den Leitungen 1c, 1d und 1e stattfindende Umpump ist ebenfalls schematisch dargestellt. Auch die in dem Nebelfänger 1b erfolgende Verdampfung der Aldehyd haltigen Reaktionsprodukte aus der Reaktionslösung ist schematisch angedeutet.

### Betriebserfahrung

Bei einer gewerblichen, industriellen Anlage zur Herstellung von Acetaldehyd aus Ethylen und Sauerstoff wurde der mit einer konventionellen keramischen Ausmauerung ausgelegte Schlaufenreaktor durch einen solchen aus Titan gefertigten ersetzt, der mit einer äußeren Wärmeisolierung aus 100 Millimetern Mineralfasern belegt wurde. Die sonstigen Apparateabmessungen wurden exakt übernommen. Als Ergebnis durch diese Maßnahme wurde innerhalb des üblichen Lastbereichs von 3,2 bis 6,1 t/h Ethylen eine Reduktion des spezifischen Abgasanfalls um 60 bis 70 % je nach Anlagenlast beobachtet. Innerhalb dieses Lastbereichs zeigte sich ebenfalls eine Abnahme des CSB-Austrags (Chemischer Sauerstoffbedarf) über das Betriebsabwasser im Durchschnitt von 0,55 t/d. Die Gesamtausbeute der Acetaldehydanlage konnte um etwa 1 %, bezogen auf Ethyleneinsatz, erhöht werden. Um die bisherige Ethylenaufnahme zu erzielen, kann die nach dem erfindungsgemäßen Verfahren betriebene Acetaldehydanlage nunmehr bei einem niedrigeren Anlagendruck gefahren werden, der um etwa 10 % geringer ist als nach der konventionellen Arbeitsweise.

## Patentansprüche

1. Einstufiges Verfahren zur Oxidation, von Ethylen zu Acetaldehyd in Gegenwart einer wässrigen Katalysatorlösung, die aus einer Lösung von Kupferchlorid und Palladiumchlorid besteht, unter Aufrechterhaltung der Umwälzung der wässrigen Katalysatorlösung in einem Schlaufenreaktor, der aus einem Reaktor und einem Nebelfänger aufgebaut ist, **dadurch gekennzeichnet, dass** der gesamte Schlaufenreaktor eine von außen aufgebrachte Wärmeisolierung aufweist.

2. Verfahren gemäß Anspruch 1, **dadurch gekennzeichnet, dass** die von außen aufgebrachte Wärmeisolierung in einer Dicke von bis zu 100 Millimetern aufgetragen wird.

3. Verfahren gemäß Anspruch 1 oder 2, **dadurch gekennzeichnet, dass** die von außen aufgebrachte Wärmeisolierung aus einem Material besteht, das bei der Reaktionstemperatur der Acetaldehydbildung eine ausreichende Temperaturbeständigkeit aufweist.

4. Verfahren gemäß Anspruch 3, **dadurch gekennzeichnet, dass** die von außen aufgebrachte Wärmeisolierung aus Mineralfasern oder Glasschaum besteht.

5. Verfahren gemäß einem oder mehreren der Ansprüche 1 bis 4, **dadurch gekennzeichnet, dass** der Druck tragende Teil des Schlaufenreaktor aus einem gegenüber der wässrigen Katalysatorlösung korrosionsbeständigen Material gefertigt ist.

6. Verfahren gemäß Anspruch 5, **dadurch gekennzeichnet, dass** das korrosionsbeständige Material ausgewählt wird aus Titan, Tantal oder Niob-Zirkon-Legierungen.

7. Verfahren gemäß einem oder mehreren der Ansprüche 1 bis 4, **dadurch gekennzeichnet dass** der Druck tragende Teil des Schlaufenreaktors aus C-Stahl oder üblichen Edelstählen gefertigt ist und gleichzeitig die Reaktorinnenwand mit einem korrosionsstabilen Material beschichtet ist, das zugleich eine ausreichende Temperaturbeständigkeit bei Reaktionstemperatur aufweist.

8. Verfahren gemäß Anspruch 7, **dadurch gekennzeichnet, dass** die korrosionsstabile und zugleich temperaturbeständige Beschichtung aus Email oder polyhalogenierten Kohlenwasserstoffen besteht.

## Claims

1. Single-stage process for the oxidation of ethylene to acetaldehyde in the presence of an aqueous catalyst solution comprising a solution of copper chloride and palladium chloride with maintenance of the circulation of the aqueous catalyst solution in a loop reactor made up of a reactor and a demister, **characterized in that** the entire loop reactor is provided with thermal insulation applied from the outside.

2. Process according to Claim 1, **characterized in that** the thermal insulation applied from the outside is applied in a thickness of up to 100 millimetres.

3. Process according to Claim 1 or 2, **characterized in that** the thermal insulation applied from the outside consists of a material which has sufficient heat resistance at the reaction temperature of acetaldehyde formation.

4. Process according to Claim 3, **characterized in that** the thermal insulation applied from the outside comprises mineral fibres or glass foam.

5. Process according to one or more of Claims 1 to 4, **characterized in that** the pressure-bearing part of the loop reactor is made of a material which is corrosion resistant in respect of the aqueous catalyst solution.

6. Process according to Claim 5, **characterized in that** the corrosion-resistant material is selected from among titanium, tantalum and niobium-zirconium alloys.

7. Process according to one or more of Claims 1 to 4, **characterized in that** the pressure-bearing part of the loop reactor is made of carbon steel or customary stainless steels and the inner wall of the reactor is at the same time coated with a corrosion-resistant material which also has sufficient heat resistance at the reaction temperature.

8. Process according to Claim 7, **characterized in that** the corrosion-resistant and also heat-resistant coating comprises enamel or polyhalogenated hydrocarbons.

## Revendications

1. Procédé à une étape pour l'oxydation d'éthylène en acétaldéhyde en présence d'une solution catalytique aqueuse, qui consiste en une solution de chlorure de cuivre et de chlorure de palladium, avec rétention de la circulation de la solution catalytique aqueuse dans un réacteur à boucle, qui est constitué d'un réacteur et d'un collecteur de brume, **caractérisé en ce que** la totalité du réacteur à boucle comporte une isolation thermique appliquée depuis l'extérieur.

2. Procédé selon la revendication 1, **caractérisé en ce que** l'isolation thermique appliquée depuis l'extérieur est appliquée en une épaisseur inférieure ou égale à 100 millimètres.

3. Procédé selon la revendication 1 ou 2, **caractérisé en ce que** l'isolation thermique appliquée depuis l'extérieur est constituée d'un matériau qui présente une résistance à la température suffisante à la température de réaction de la formation de l'acétaldéhyde.

4. Procédé selon la revendication 3, **caractérisé en ce que** l'isolation thermique appliquée depuis l'extérieur est constituée de fibres minérales ou de mousse de verre.

5. Procédé selon une ou plusieurs des revendications 1 à 4, **caractérisé en ce que** la partie qui supporte la pression du réacteur à boucle est fabriquée en un matériau résistant à la corrosion de la solution catalytique aqueuse.

6. Procédé selon la revendication 5, **caractérisé en ce que** le matériau résistant à la corrosion est choisi parmi le titane, le tantale ou les alliages niobium-zirconium.

7. Procédé selon une ou plusieurs des revendications 1 à 4, **caractérisé en ce que** la partie qui supporte la pression du réacteur à boucle est fabriquée en acier au carbone ou en des aciers inoxydables usuels et simultanément la paroi interne du réacteur est enduite d'un matériau stable à la corrosion qui présente en même temps une résistance à la température suffisante à la température de réaction.

8. Procédé selon la revendication 7, **caractérisé en ce que** le revêtement stable à la corrosion et en même temps résistant à la température est constitué d'émail ou d'hydrocarbures polyhalogénés.
